# FASCICULE DE BREVET EUROPEEN

(11) **EP 0 910 341 B1**
(45) Date de publication et mention de la délivrance du brevet: **30.06.2004**
(21) Numéro de dépôt: 97934568.3
(22) Date de dépôt: 11.07.1997
(51) Int. Cl.: A61K 9/00

(54) **COMPOSITION PHARMACEUTIQUE DESTINEE A UNE ADMINISTRATION PAR VOIE BUCCALE**
ARZNEIMITTEL ZUR ANWENDUNG IM MUND
PHARMACEUTICAL COMPOSITION TO BE ADMINISTERED ORALLY

(30) Priorité: 12.07.1996 FR 9608740
(43) Date de publication de la demande: 28.04.1999
(73) Titulaire: Maurel Santé, 34160 Castries-l'Amaury (FR); Laboratoires Thelfar, 30700 Uzès (FR)
(72) Inventeur: MAUREL, Jean-Claude, F-78490 Montfort l'Amaury (FR); SANCY, Yolande, F-30000 Nîmes (FR)
(74) Mandataire: Giraud, Françoise
(86) Numéro de dépôt international: PCT/FR1997/001277
(87) Numéro de publication internationale: WO 1998/002140

(56) Documents cités:
- EP-A- 0 449 782
- US-A- 5 462 749
- DATABASE WPI Week 8122 Derwent Publications Ltd., London, GB; AN 81-002304 XP002042734 & RO 70 142 A (TERAPIA INTR. MED.) , 30 août 1980
- DATABASE WPI Section Ch, Week 7813 Derwent Publications Ltd., London, GB; Class B,Page 05, AN 78-23853 XP002042735 & JP 50 157 517 A (ONO PHARM. CO. LTD.) , 19 décembre 1975
- DATABASE WPI Section Ch, Week 8213 Derwent Publications Ltd., London, GB; Class B,Page 04, AN 82-25217 XP002042736 & JP 57 032 221 A (MORINAGA & CO.) , 20 février 1982
- DATABASE WPI Section Ch, Week 9642 Derwent Publications Ltd., London, GB; Class B,Page 07, AN 96421887 XP002042737 & JP 08 208 456 A (DAIKYO YAKUHIN KOGYO) , 13 août 1996

## Description

La présente invention concerne une nouvelle composition pharmaceutique destinée à une administration par voie buccale avec absorption d'au moins un principe actif à travers les muqueuses de la bouche.

Elle concerne essentiellement de nouvelles formulations galéniques destinées à une absorption préférentiellement par voie per-linguale ou sub-linguale pouvant être utilisées quelle que soit la substance médicamenteuse concernée.

Beaucoup de substances médicamenteuses sont altérées ou détruites dans le tube digestif, en particulier dans l'estomac, mais aussi dans le duodénum ou l'intestin grêle par les différents sucs digestifs; de plus les substances absorbées par voie digestive subissent un premier passage hépatique, et donc une première métabolisation avant de rejoindre les tissus périphériques.

Les formes gastro-résistantes permettent de pallier à la destruction dans l'estomac, mais pas aux autres difficultés que nous venons d'énumérer.

La voie sublinguale (ou per muqueuse) est la voie d'administration idéale pour toutes les substances médicamenteuses qui sont altérées par le tube digestif lors de sa traversée, ou encore inactivées par le premier passage hépatique; en effet, cette voie permet un premier passage dans la grande circulation et une distribution tissulaire périphérique complète avant le premier passage hépatique. Enfin l'absorption est beaucoup plus rapide et permet lorsque cela est nécessaire, en particulier dans le traitement des douleurs ou des spasmes, d'avoir une efficacité thérapeutique plus rapide.

Cette voie d'administration est la seule qui permette d'obtenir une efficacité thérapeutique avec des substances fragiles telles que: les extraits végétaux, certaines vitamines...

Il va de soi que ces formulations sont tout aussi adaptées à toutes les molécules chimiques actives comme médicament, quelle que soit leur stabilité dans le tube digestif.

Les difficultés d'une formulation galénique permettant une absorption dans la bouche, per-muqueuse et sublinguale sont multiples:
- s'il s'agit d'une forme liquide, le problème majeur est la stabilité de la solution en l'absence d'alcool; or la présence d'alcool interdit toute consommation par des enfants; de plus, les formes liquides sont d'utilisation malaisée.
- s'il s'agit d'une forme solide, il faut disposer d'un comprimé , d'une tablette ou de toute autre forme qui se délite facilement dans la bouche, afin de libérer aisément la substance médicamenteuse, tout en présentant une dureté satisfaisante dans son emballage. Il faut également que le produit permette d'introduire une quantité significative de la substance active. Il faut, en plus, cacher le goût souvent très désagréable du produit et, enfin, obtenir une parfaite stabilité dans le temps de la substance active. Lors de la fabrication, il faut que le mélange des substances soit parfaitement homogène et éviter toute montée en température susceptible d'altérer les principes actifs dont certains, tels les extraits végétaux, sont particulièrement sensibles à la chaleur.

Il existe sur le marché du médicament deux types de produits destinés à une absorption buccale:
- les produits à action locale: pastilles ou comprimés à sucer ou à croquer, avec une activité locale, antiseptique, antibiotique, anti-inflammatoire ou antalgique, dans la bouche ou dans la gorge,
- les produits de type comprimés sub-linguaux à visée thérapeutique générale.

On distingue, en fonction de leur composition, trois types de produits à action locale:
- les comprimés ou tablettes dont la base excipiendaire est constituée par des sucres (saccharose, lactose, sirop de glucose, sorbitol) auxquels sont associés des liants et lubrifiants permettant la compression (stéarate de magnésium, silice colloïdale, carboxyméthylcellulose calcique, amidon...) et qui sont ensuite aromatisés et éventuellement enrobés; il peut s'agir aussi de pastilles à base de sucres cuits,
- les pastilles ou pâtes à sucer dont la base excipiendaire est constitué d'un agent gélifiant, en particulier gomme arabique, à laquelle est associée un ou plusieurs sucres. Ces produits sont généralement fabriqués en milieu aqueux,
- les produits dont l'excipient est constitué de sucres modifiés permettant une compression, les plus utilisés de ces sucres étant les maltodextrines.

En ce qui concerne les produits à action générale, leurs bases exeipiendaires sont généralement constituées de:
- de gommes ou de mélanges de gommes et d'alcools gras, ou
- de lactose, saccharose, cellulose micro-cristalline, stéarate de magnésium ou silice colloïdale. Il est souvent ajouté à la formule un constituant tel que la polyvinylpyrrolidone ou la polyplasdone qui s'hydrate en présence d'eau et provoque la désagrégation du comprimé.

Dans toutes les formes galéniques utilisées actuellement, on constate que :
- soit le produit est sec et simplement comprimé (formulation à base de sucre ou de sucres modifiés). Il est alors difficile d'incorporer à l'excipient une masse supérieure à 10 % de la masse totale sans perte de dureté du produit fini. Cette formulation n'est utilisable qu'avec des substances ayant des caractéristiques physiques adaptées, mais surtout parfaitement stables en toutes circonstances, car un enrobage de protection est difficile du fait de l'extrême fragilité du produit fini; enfin il est très difficile de cacher les goûts désagréables des principes actifs utilisés en dépit des aromatisations,
- soit le produit est une gomme avec des formulations diverses associées: la difficulté est liée à la présence d'eau en quantité importante qui est un obstacle à la stabilité de beaucoup de substances médicamenteuses. C'est le cas des extraits végétaux mais aussi de l'acide acétylsalicylique par exemple.

De plus, le produit fini obtenu ne se délite pas dans la bouche, comme il est souhaitable pour un médicament sublingual.

Les inventeurs ont maintenant découvert une nouvelle formulation qui permet de pallier l'ensemble des inconvénients qui existent avec les formulations actuelles, en ajoutant, dans certaines conditions de fabrication, une huile ou graisse, de préférence végétale naturelle, mais aussi tout produit apportant des lipides, tels que le lait animal ou végétal, en quantité relative importante, de l'ordre de 10 % en moyenne, aux constituants habituels de ce type de formulation; elle permet de lier les différents constituants sans que la pâte obtenue ne durcisse dans le temps et apporte au mélange une texture qui se délite facilement en bouche; enfin elle permet une parfaite homogeité de la composition et une stabilité des constituants, en particulier des principes actifs du médicament; La matrice est constituée d'un mélange homogène et intime des différents constituants : cette nature de la matrice est différente de l'enrobage d'un noyau de sucre par une encapsulation grasse comme décrit dans le brevet EP 0 449 782 A1: ce produit est très différents car multicouche « multiphase compressed tablet » ( p.2, ligne 38-39, ligne 49-52; p.3, ligne 9-11,..., fig.3, fig.4..).

Cette formulation permet également de solubiliser ou de mettre en suspension des principes actifs hygroscopiques ou insolubles en milieu aqueux.

Ainsi, selon l'une de ses caractéristiques essentielles, l'invention concerne une composition pharmaceutique destinée à une administration par voie buccale avec absorption d'au moins un principe actif à tout niveau du tube digestif, mais de façon préférentielle à travers les muqueuses de la bouche, comprenant une base excipiendaire contenant au moins un sucre ou un agent sucrant, et éventuellement un agent de texture,
caractérisée en ce que ladite base excipiendaire est sous la forme d'un mélange intime et homogène de ses constituants et contient en outre de 1 à 15 % en masse de matière grasse d'origine végétale sous forme d'huile et/ou de graisse végétale.

Pour la préparation des compositions pharmaceutiques de l'invention, on pourra avoir recours à toute huile ou graisse pharmaceutiquement acceptable.

La fonction de cette graisse ou de cette huile, comme cela résulte des essais réalisés pour la mise au point de la présente invention, est de permettre de lier les différents constituants de ladite composition sans que la pâte obtenue ne durcisse dans le temps et de conférer au mélange une texture telle que la composition se délite facilement dans la bouche.

Les quantités relatives des différents constituants de la composition pharmaceutique selon l'invention varient largement en fonction des caractéristiques physiques de la substance active introduite, en particulier de ses caractéristiques d'hygroscopie, et de sa concentration. On comprendra donc aisément qu'il est nécessaire d'effectuer une mise au point pour chaque formulation afin de définir les quantités relatives des constituants permettant d'obtenir une pâte aux caractérisiques physiques satisfaisantes et cela est vrai, en particulier, pour la quantité d'huile ou de graisse qui, comme on l'a vu précédemment, joue un rôle essentiel dans la composition.

L'huile ou la graisse est introduite dans des proportions comprises entre 1 et 15 % en poids par rapport au poids total de la composition.

Même si on peut utiliser comme matière grasse de type huile ou graisse, toute huile compatible avec une utilisation pharmaceutique, on utilise de préférence une huile ou une graisse d'origine végétale, de préférence hydrogénée.

Les huiles et graisses de coprah, de palme et de soja, et leurs mélanges, sont tout particulièrement préférées selon l'invention.

On peut également utiliser avantageusement du lait entier ou sa crème, et plus particulièrement du lait concentré entier qui contient en moyenne 26 à 30 % de lipides en poids sec; il permet en particulier de tamponner l'acidité de certains principes actifs.

On peut encore utiliser un lait d'origine végétale tel que le lait de soja, ou encore le beurre de cacao.

Selon un autre avantage, l'invention permet de réaliser des compositions solides pouvant contenir des quantités importantes de produits actifs, en particulier jusqu'à 20 % en poids de produit actif sans avoir recours à des quantités importantes d'eau comme cela est le cas selon l'art antérieur.

Ainsi, selon l'une de ses caractéristiques, l'invention concerne une composition qui contient une quantité maximale d'eau non liée inférieure à 15 % en poids par rapport au poids total de la composition. Ceci s'avère particulièrement avantageux puisqu'une telle formulation permet d'éviter toute altération des principes actifs du médicament.

Comme on l'a vu précédemment, les compositions de l'invention ont pour caractéristique essentielle de contenir, en plus des constituants de base classiquement utilisés dans les compositions pharmaceutiques à administration par voie buccale avec absorption à travers les muqueuses de la bouche, au moins un produit de type huile ou graisse conférant à la base excipiendaire des caractéristiques structurales permettant son délitement rapide au niveau de la bouche. Les différents constituants autres de la composition peuvent varier dans de larges proportions. Toutefois, les compositions de l'invention contiennent avantageusement de 50 à 90 % en poids d'au moins un sucre et de 0 à 10 % en poids d'au moins un produit agissant en tant qu'agent de texture.

Les sucres utilisés dans les compositions de l'invention sont avantageusement choisis parmi la famille constituée du saccharose, des polyoses, des trioses, du maltose, du dextrose, du fructose, du lactose et des sucres modifiés, en particulier des maltodextrines ainsi que de leurs mélanges, ou encore des agents sucrants comme les polyols et les polydextroses.

Les agents de texture éventuellement utilisés sont ceux classiquement utilisés dans le type d'application visé selon l'invention. Il s'agit en particulier des alginates, de la pectine, de la gélatine, des carraghénanes, des protéines de lait, de la fécule, des amidons modifiés, des amandons de fruits à noyau, une gomme, en particulier la gomme arabique, et de leurs mélanges.

Un des avantages particuliers des formulations galéniques selon l'invention est qu'elles permettent l'incorporation d'un nombre important de principes actifs. Ces principes actifs sont avantageusement des produits qui se présentent sous forme de poudre à l'état sec. Toutefois, les principes actifs peuvent être également choisis parmi les liquides, en particulier parmi les liquides lipidiques. Ceci est rendu tout particulièrement possible du fait de la présence d'une quantité relativement importante de matières grasses dans la composition de l'invention.

Les principes actifs peuvent être de nature très variée. Il peut, en particulier, s'agir de molécules chimiques, d'extraits végétaux, de vitamines, d'oligo-éléments .

On notera qu'un avantage tout particulier des compositions galéniques de l'invention est qu'elles permettent l'utilisation de proportions allant jusqu'à 30 % de la masse de la composition en principes actifs.

Bien entendu, les compositions pharmaceutiques selon l'invention peuvent inclure en outre, comme cela est classique dans le domaine, différents additifs, tels que des arômes, des colorants, des produits d'enrobage.

Les produits de l'invention sont généralement présentés sous forme de tablettes, de pâtes, de pastilles à croquer ou à sucer.

Un des avantages tout particulier des compositions de l'invention est qu'elles peuvent être obtenues dans un procédé particulièrement simple présentant l'avantage de pouvoir être mis en oeuvre sans un chauffage supérieur à environ 40°C pour les formes obtenues par laminage, ou de 40° à 50°C pour les formes coulées, ce qui permet d'utiliser, sans les altérer, des molécules en fonction de leur thermolabilité. Les grandes étapes du procédé de fabrication des produits selon l'invention sont résumées ci-dessous:

### Etape 1

### Mélange des ingrédients:

Dans un premier temps on introduit les produits sous forme de poudre, y compris les principes actifs des médicaments, puis éventuellement les agents de texture , les lipides et les liquides (sirop, eau...). Le mélange est agité pendant une durée et avec une vitesse qui dépend des quantités relatives des différents constituants. Dans une variante de l'invention, on introduit en premier lieu les liquides (sirop et éventuellement agents de texture) puis les principes actifs et les lipides; le choix de la méthode est conditionné par les caractéristiques physico-chimiques du principe actif. Dans certains cas, en particulier lorsque les principes actifs du médicament ont une forte hygroscopie, ils seront introduits après mélange dans l'huile ou la graisse, alors que la pâte est encore très souple, à une température de l'ordre de 30°C à 50°C, dans un pétrin à double enveloppe ou un mélangeur adapté à cette fonction, selon la formulation mise en oeuvre.

### Etape 2 : formes obtenues par laminage

Laminage ou mise sous presse ;
La quantité de matière sèche de la pâte est de 90 ± 5 %;
Cette étape est éventuellement précédée d'une étape d'extrusion sur tapis;
Puis découpage.

### Etape 2 bis : formes obtenues par coulage

Le mélange contient environ 80 ± 5 % de matières sèches, et se trouve au moment du coulage à une température comprise entre 40° et 60°C.

Le coulage est effectué notamment dans des moules d'amidon pré-formés.

Elimination de l'amidon par soufflage.

Après séchage, le produit fini contient 90 ±5% de matières sèches.

### Etape 3

Vernissage à base d'huile et de cire ou sucrage et dragéification éventuelle, tant pour une meilleure stabilité dans le temps du médicament obtenu, que pour la présentation visuelle du produit fini.

La dragéification peut être réalisée à partir de matière sucrée, et le vernissage final à partir d'huile végétale et de cire de Camauba ou de tout autre méthode habituelle.

### Etape 4

Conditionnement primaire éventuel, sous blisters, en sachets ou en boites du produit fini obtenu après l'étape 2 ^{bis} ou l'étape 3..

Ainsi, l'invention concerne des formulations qui permettent de surmonter simultanément tous les inconvénients des formulations connues à ce jour. Les avantages sont résumés ci-dessous:
- Elle permet un mélange homogène avec les principes actifs, et donc une parfaite répartition unitaire lors de la fabrication.
- La pâte, 1a pastille ou la tablette obtenue comme produit fini se délite rapidement dans la bouche et permet une absorption très rapide à travers la muqueuse buccale, en particulier sublinguale.
- La pâte sucrée obtenue cache parfaitement l'amertume ou les goûts désagréables des principes actifs utilisés, et permet une prise par les enfants; ainsi cette forme est la seule qui permet de proposer à des enfants à partir de 3 ans des traitements par des extraits végétaux per os.
- La très faible quantité d'eau libre utilisée pour la préparation (moins de 10 %dans les formes par laminage et 20 % pour les formes coulées) évite d'altérer les principes actifs des médicaments.
- La présence simultanée d'eau et de lipides permet de fabriquer des médicaments dont les principes actifs peuvent aussi bien être hydrosolubles que liposolubles.
- On peut incorporer jusqu'à 30 % de la masse en principes actifs.
- Lors des étapes de la fabrication des formes par laminage, aucun chauffage supérieur à 40°C n'intervient, ce qui permet d'utiliser sans les altérer des molécules thermolabiles; Dans les formes coulées, la température comprise entre 40° et 50°C ne dure que peu de temps et reste peu aggressive pour les molécules thermo-labiles.

La formulation est stable et permet d'obtenir une excellente stabilité dans le temps du médicament obtenu.

### EXEMPLES

Les pourcentages donnés dans les exemples ci-dessous sont des pourcentages en poids avant dragéification éventuelle.

### Exemple 1

solution de saccharose, sirop de glucose, dextrose ( en solution à 80%) 28 %
sucre glace amylacé 39 % maltodextrine 10 %
gélatine (solution à 40%) 4 %
graisse végétale hydrogénée (telle que palme et/ou soja) 8 %
extrait sec de Reines des Près (Spiraea ulmaria) 10%
colorant, arôme citron vert.
laminage puis découpage;
tablette de 2 g.
indication thérapeutique et posologie:
   - états fabriles et états grippaux:
   - chez l'adulte, croquer une tablette toutes les 3 heures, prendre 4 tablettes par jour.
   - chez l'enfant, une tablette par jour par quinze kilos de poids.

### Exemple 2

solution de saccharose, sirop de glucose (à 82 %) 39 %
sucre glace amylacé 28 %
maltodextrine 5 %
gomme arabique ( spray gum) 9 %
huile végétale (Palme) 9 %
extrait sec de Mélisse (Melissa officinalis) 10 %
eau qsp, arôme citron, menthe, colorant.
laminage puis découpage
indication thérapeutique et posologie:
   - lenteur digestive, lourdeur, ballonnements, éructations.
   - chez l'adulte, croquer une tablette toutes les 3 heures, prendre 4 tablettes par jour.
   - chez l'enfant, une tablette par jour par quinze kilos de poids.

### Exemple 3

sirop de maltitol 67 %
gélatine (solution à 33%) 18 %
huile végétale 5 %
paracetamol (200 mg par comprimé) 10 %
arôme orange, colorant.
   Agitation forte pouvant aller jusqu'à l'aération pour alléger la pâte. Cette formulation est intéressante pour les principes actifs insolubles dans l'eau ou hygroscopiques, car ils sont mis et maintenus en suspension de façon homogène. indications thérapeutiques: traitement symptomatique des affections douloureuses ou fébriles, des algies d'origine diverses.

### Exemple 4

sirop de saccharose, sirop de glucose (à 75% de matières sèches) 57 %
lait condensé entier 8 %
graisse végétale 2 %
poudre de cacao 3%
sel de calcium 30 %
vitamine D 3 400 UI par tablette
arôme orange, colorant.
coulage à la température de 40° à 45°C;
indications thérapeutiques: osteoporose.

### Exemple 5

saccharose, polyoses, maltotrioses, maltose, saccharose, dextrose (en solution à 75%) 85 %
lait entier condensé 8,5%
ibuprofène micro encapsulé 6,5 %
colorant, arôme caramel
coulage à 40° / 45°C ;
indications thérapeutiques: douleurs d'origine diverses, fièvre.
posologie chez l'enfant : croquer une tablettes en une seule prise; prendre 2 à 4 tablettes par jour en fonction de l'âge si les douleurs persistent.

## Revendications

1. Composition pharmaceutique destinée à une administration par voie buccale avec absorption d'au moins un principe actif à tout niveau du tube digestif, mais de façon préférentielle à travers les muqueuses de la bouche, comprenant une base excipiendaire contenant au moins un sucre et/ou au moins un agent sucrant et éventuellement un agent de texture, **caractérisée en ce que** ladite base excipiendaire est sous la forme d'un mélange intime et homogène de ses constituants et contient en outre de 1 à 15 % en masse de matière grasse d'origine végétale sous forme d'huile et/ou de graisse végétale.

2. Composition selon la revendication 1, **caractérisée en ce que** ladite matière grasse d'origine végétale est une huile ou une graisse d'origine végétale hydrogénée.

3. Composition selon l'une des revendications 1 ou 2 , **caractérisée en ce que** ladite matière grasse végétale est une huile de coprah , de palme/palmiste ou de soja, ou un mélange de ces huiles, ou encore un lait ou un beurre végétal.

4. Composition selon l'une des revendications 1 à 3 , **caractérisée en ce que** ladite base contient une quantité maximale d'eau non liée représentant moins de 10 % en poids par rapport au poids total du produit fini.

5. Composition selon l'une des revendications 1 à 4 , **caractérisée en ce que** le(les) sucre(s) et l'(les) agent(s) sucrant(s) contenu(s) dans ladite base est(sont) dans une proportion pondérale comprise entre 50 et 90 % par rapport au poids total de ladite composition.

6. Composition selon l'une des revendications 1 à 5, **caractérisée en ce que** lesdits sucres ou agents sucrants sont choisis dans la famille du saccharose des polyoses, des trioses, du maltose, du dextrose, du fructose, du lactose et des sucres modifiés et de leurs mélanges, ainsi que des agents sucrants tels que les polyols et les polydextroses.

7. Composition selon la revendication 6, **caractérisée en ce que** ledit agent sucrant est un polyol.

8. Composition selon l'une des revendications 1 à 7, **caractérisée en ce qu'**elle contient de 0 à 15 % en poids par rapport au poids total de ladite composition d'agent de texture.

9. Composition selon l'une des revendications 1 à 8, **caractérisée en ce que** l'agent de texture est choisi dans le groupe comprenant les alginates, la pectine, la gélatine, les carraghénanes, les protéines de lait, la fécule, les amidons modifiés, les amandons de fruits à noyau, une gomme, en particulier la gomme arabique et leurs mélanges.

10. Composition selon l'une des revendications 1 à 9, **caractérisée en ce que** le(s) principe(s) actif(s) est (sont) sous la forme d'une poudre ou d'un liquide.

11. Composition selon l'une des revendications 1 à 10, **caractérisée en ce qu'**elle contient en outre un arôme et/ou un colorant et/ou un produit d'enrobage.

12. Composition selon l'une des revendications 1 à 11, **caractérisée en ce qu'**elle est sous forme de tablettes, de pâtes, de pastilles à croquer.

13. Procédé pour favoriser l'absorption par voie buccale d'un produit choisi parmi les vitamines et les oligo-éléments, **caractérisé en ce qu'**il consiste à incorporer ledit produit dans une base excipiendaire telle que définie dans l'une des revendications 1 à 9.

## Patentansprüche

1. Arzneimittel zur Anwendung im Mund mit Absorption von mindestens einem Aktivprinzip auf jeder Höhe des Verdauungskanals, allerdings auf bevorzugte Weise durch die Schleimhäute des Mundes, umfassend eine Bindemittelbasis, die mindestens einen Zucker und/oder mindestens einen Süßstoff und eventuell ein Strukturmittel enthält, **dadurch gekennzeichnet, dass** die Bindemittelbasis in Form einer engen und homogenen Mischung ihrer Bestandteile vorhanden ist und außerdem 1 bis 15 Massenprozent fettigen Materials pflanzlichen Ursprungs in Form eines Öls und/oder pflanzlichen Fettes enthält.

2. Arzneimittel nach Anspruch 1, **dadurch gekennzeichnet, dass** die Fette pflanzlichen Ursprungs ein Öl oder ein Fett wasserstoffhaltigen pflanzlichen Ursprungs sind.

3. Arzneimittel nach einem der Ansprüche 1 oder 2, **dadurch gekennzeichnet, dass** das pflanzliche Fett ein Kopra-, Palmen-/Palmenkohl- oder Sojaöl oder eine Mischung dieser Öle oder auch eine Pflanzenmilch oder -butter ist.

4. Arzneimittel nach einem der Ansprüche 1 bis 3, **dadurch gekennzeichnet, dass** die Basis eine nicht gebundene maximale Wassermenge enthält, die weniger als 10 Gew.-% im Verhältnis zum Gesamtgewicht des Endproduktes ausmacht.

5. Arzneimittel nach einem der Ansprüche 1 bis 4, **dadurch gekennzeichnet, dass** der (die) Zucker und der (die) Süßstoff(e), der (die) in der Basis enthalten ist (sind), in einem Gewichtsverhältnis zwischen 50 und 90 % in Bezug auf das Gesamtgewicht des Arzneimittels vorhanden ist (sind).

6. Arzneimittel nach einem der Ansprüche 1 bis 5, **dadurch gekennzeichnet, dass** die Zucker oder Süßstoffe in der Familie der Saccharose, Polyosen, Triosen, Maltose, Dextrose, Fruktose, Laktose und der modifizierten Zuckerarten und ihrer Mischungen, sowie der Süßstoffe, wie beispielsweise der Polyole und der Polydextrosen, ausgewählt werden.

7. Arzneimittel nach Anspruch 6, **dadurch gekennzeichnet, dass** der Süßstoff ein Polyol ist.

8. Arzneimittel nach einem der Ansprüche 1 bis 7, **dadurch gekennzeichnet, dass** es 0 bis 15 Gew.-% eines Strukturmittels im Verhältnis zum Gesamtgewicht des Arzneimittels enthält.

9. Arzneimittel nach einem der Ansprüche 1 bis 8, **dadurch gekennzeichnet, dass** das Strukturmittel in der Gruppe, die die Alginate, das Pektin, Gelatin, die Carragehnane, die Milchproteine, die Stärke, die modifizierten Stärken, die Kerne von Steinfrüchten, ein Gummi, insbesondere Arabisches Gummi, und ihre Mischungen enthält.

10. Arzneimittel nach einem der Ansprüche 1 bis 9, **dadurch gekennzeichnet, dass** das (die) Aktivprinzip(ien) in Form eines Pulvers oder einer Flüssigkeit vorhanden ist (sind).

11. Arzneimittel nach einem der Ansprüche 1 bis 10, **dadurch gekennzeichnet, dass** es ferner ein Aroma und/oder einen Farbstoff und/oder ein Umhüllungsmittel enthält.

12. Arzneimittel nach einem der Ansprüche 1 bis 11, **dadurch gekennzeichnet, dass** es in Form von Tabletten, Pasten, Kaupastillen vorhanden ist.

13. Verfahren zur Begünstigung der Absorption eines Produktes, das unter den Vitaminen und den Oligoelementen ausgewählt wird, im Mund, **dadurch gekennzeichnet, dass** es darin besteht, das Produkt in eine Bindemittelbasis, wie in einem der Ansprüche 1 bis 9 definiert, zu integrieren.

## Claims

1. A pharmaceutical composition intended for an administration *via* the buccal route with absorption of at least one active principle at any level of the digestive tract, but preferentially through the mucous membranes of the mouth, comprising an excipient base containing at least one sugar and/or at least one sweetening agent and optionally a texturing agent, **characterised in that** said excipient base is in the form of a homogeneous and intimate mixture of its constituents and further contains 1 to 15 % by weight of fatty material of vegetable origin in the form of a vegetable oil and/or vegetable fat.

2. The composition according to claim 1, **characterised in that** said fatty material of vegetable origin is a hydrogenated oil or fat of vegetable origin.

3. The composition according to one of claims 1 or 2, **characterised in that** said vegetable fatty material is a coconut oil, a palm oil/palm tree oil or a soya oil, or a mixture of these oils, or even a vegetable milk or butter.

4. The composition according to one of claims 1 to 3, **characterised in that** said base contains a maximum amount of unbound water representing less than 10% by weight with respect to the total weight of the finished product.

5. The composition according to one of claims 1 to 4, **characterised in that** the sugar(s) and the sweetening agent(s) contained in said base is (are) in a weight proportion between 50 and 90% with respect to the total weight of said composition.

6. The composition according to one of claims 1 to 5, **characterised in that** said sugars or sweetening agents are selected from the family of sucrose, polyoses, trioses, maltose, dextrose, fructose, lactose and modified sugars and mixtures thereof, as well as sweetening agents such as polyols and polydextroses.

7. The composition according to claim 6, **characterised in that** said sweetening agent is a polyol.

8. The composition according to one of claims 1 to 7, **characterised in that** it contains 0 to 15% by weight of a texturing agent with respect to the total weight of said composition.

9. The composition according to one of claims 1 to 8, **characterised in that** the texturing agent is selected from the group consisting of alginates, pectin, gelatine, carrageenans, milk proteins, starch, modified starches, stone fruit nuts, a gum, particularly gum arabic, and mixtures thereof.

10. The composition according to one of claims 1 to 9, **characterised in that** the active principle(s) is (are) in the form of a powder or a liquid.

11. The composition according to one of claims 1 to 10, **characterised in that** it further contains a flavour and/or a colorant and/or a coating product.

12. The composition according to one of claims 1 to 11, **characterised in that** it is in the form of tablets, pastes, or lozenges.

13. A method for promoting the absorption via the buccal route of a product selected from the group consisting of vitamins and trace elements, **characterized in that** it consists in incorporating said product in an excipient base as defined in one of claims 1 to 9.
